# EUROPEAN PATENT APPLICATION

(11) **EP 4 140 505 A1**
(43) Date of publication of application: **01.03.2023**
(21) Application number: 21193360.1
(22) Date of filing: 26.08.2021
(51) Int. Cl.: A61K 49/06

(54) **PURIFIED SIGNAL-ENHANCED CONTRAST AGENTS FOR MAGNETIC RESONANCE IMAGING**

(71) Applicant: Max-Planck-Gesellschaft zur Förderung der Wissenschaften e.V., 80539 München (DE)
(72) Inventor: Glöggler, Stefan, 37070 Göttingen (DE); Korchak, Sergey, 37070 Göttingen (DE)
(74) Representative: Schulz Junghans Patentanwälte PartGmbB

(57) **Abstract**

The present invention relates to improved methods for obtaining purified contrast agents that are suitable for magnetic resonance imaging. The contrast agents are prepared by a method such dynamic nuclear polarization (DNP), hydrogenative parahydrogen induced polarization (PHIP), or Signal Amplification By Reversible Exchange (SABRE). High degrees of purity are achieved by performing an evaporation step to separate a signal enhanced precursor or the contrast agent from a metal catalyst or a source of radicals.

## Description

The present invention relates to improved methods for obtaining purified contrast agents that are suitable for magnetic resonance imaging. The contrast agents are prepared by a method such as dynamic nuclear polarization (DNP), hydrogenative parahydrogen induced polarization (PHIP), or Signal Amplification By Reversible Exchange (SABRE). High degrees of purity are achieved by performing an evaporation step to separate a signal enhanced precursor or the contrast agent from a metal catalyst or a source of radicals.

### Background of the Invention

Magnetic resonance imaging (MRI) is a widely used technique in research institutions and clinics to examine and diagnose disease patterns. In order to make diseases more visible, contrast agents are injected into the organism. Approximately 30 million examinations with contrast agents are performed in clinics worldwide every year.

A special form of contrast agents that allows chemical processes, such as metabolism, to be observed directly is based on the process of hyperpolarization. In the field of nuclear magnetic resonance (NMR), the phenomenon on which MRI is based, hyperpolarization procedures increase the signals of contrast agents by several orders of magnitude. Hyperpolarization is used, for example, to increase the signals of metabolites, which are then injected to visualize the metabolism in tumors. This has already been shown in clinical studies in humans. In particular, it has been shown that such a method can be used to identify tumors and assess the effectiveness of treatments. In future it has potential to replace positron emission tomography (PET) in staging of the cancer.

The common method of amplifying signals by hyperpolarization is based on the technique of dynamic nuclear polarization (DNP). In order to achieve the greatest possible signal amplification, contrast agents are usually hyperpolarized at cryogenic temperatures (i.e. at temperatures below 2K) in the presence of radicals using microwave radiation. This process takes several tens of minutes to hours and is therefore very time-consuming, which severely limits the use of this method. After signal amplification, the contrast agents are heated and dissolved so that they can be injected whereby the radicals are typically filtered.

Another method of hyperpolarization, which delivers amplified signals within a few seconds, is based on parahydrogen or ortho-deuterium. The use of parahydrogen is the more common form of hyperpolarization. Parahydrogen is a spin isomer of hydrogen gas which is enriched by passing the corresponding gas at low temperatures over a catalyst (usually iron oxide or activated carbon). At 77K, a degree of enrichment of about 50% is achieved and at 25K this is about 100%. If the catalyst is removed after enrichment, the gas can be filled into bottles and stored at room temperature for days to weeks. In parahydrogen-based hyperpolarization processes, the spin order generated by the enrichment of parahydrogen is converted into amplified signals from molecules, which are then used as contrast agents.

There are two modalities in which parahydrogen can be coupled with the substrate in order to achieve a hyperpolarized signal. On the one hand, parahydrogen can be added to a molecule by means of a catalyst, which changes the chemical structure of the substrate (hydrogenative parahydrogen induced polarization, PHIP). After the addition process the spin order is converted into an amplified, observable signal. On the other hand, parahydrogen can form a temporarily stable complex with a catalyst and a compound of interest (e.g. metabolites), in which the spin order is converted into amplified signals of the compound of interest. The labile complex then separates in its components again and an unchanged, signal-amplified contrast agent is obtained. This non hydrogenative PHIP method is called SABRE (Signal Amplification By Reversible Exchange, SABRE).

Overall, pyruvate, lactate, acetate, succinate, fumarate, acetoacetate and hydroxybutyrate and potential alkylesters thereof are the most important contrast agents. Although these molecules can be signal-enhanced via PHIP or SABRE, either by producing a precursor with an unsaturated moiety such as vinyl ester that is hydrogenated with para-hydrogen and subsequently transformed into the metabolite or directly used as ester (PHIP) or via the potential direct signal-enhancement via SABRE, a catalyst is always required to produce enhanced signals. When the contrast agent is produced by DNP, a source for generating radicals is required.

This catalyst is however hard to separate and usually consists of a heavy metal such as rhodium, iridium, platinum, palladium, ruthenium and rhenium. Similarly, sufficient separation of the source of radicals is difficult to achieve.

So far, purification procedures rely on filtration, precipitation and washing or liquid phase separation of an organic solvent and water to deliver an aqueous injection solution.

All these methods have shown that it is hard to filter out e.g. the last amounts of a rhodium catalyst. The rhodium content is usually reduced by a factor of 100-1000 by these state-of-the-art techniques leaving micromolar concentrations of metal content behind. Assuming a 25 mL injection dose which is typical in clinical studies (C. Y. Lee et al. Neuroimage 204, 116202, 2020) using the DNP technology, this would result in an injection of e.g. 10 µg rhodium (5 µM metal concentration in 25 mL injection solution, 103 g/mol molecular weight of rhodium). In comparison, these values are about 1000-fold higher than the metal base levels found in human blood as suggested by recent studies (G. Rentschler et al. International Journal of Hygiene and Environmental Health 221, 223-230, 2018).

For a better removal of catalyst or the source of radicals, the purification described here will take place via a quick evaporation/distillation step. Whereby evaporation can also mean to use a stripping gas. The evaporation with or without stripping gas can be facilitated by applying a vacuum. The stripping gas can be any gas or steam, preferably inert gas e.g. nitrogen, hydrogen. The stripping gas is more efficient combined with vacuum.

This evaporation step has proven to reduce the metal content by another factor of 100-1000 compared to the state-of-the-art purification as confirmed by ICP-MS analysis. For example, the rhodium content is hence in the nanomolar concentration range.

Compared to other methods, the approach presented here is in particular suitable for the large-scale production of hyperpolarized contrast agents with applicability in clinics.

Based on the above-mentioned state of the art, the objective of the present invention is to provide means and methods to purify a signal enhanced compound more efficiently compared to methods of the state of the art. This objective is attained by the subject-matter of the independent claims of the present specification, with further advantageous embodiments described in the dependent claims, examples, figures and general description of this specification.

### Summary of the Invention

The invention relates to a method for preparing a contrast agent characterized in that the method comprises an evaporation step, wherein a signal enhanced compound that comprises a nuclear spin is separated from a liquid.

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of' or "consisting of."

Where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

The term "alkyl" in the context of the present specification relates to a saturated or unsaturated linear or branched hydrocarbon. For example, a *C₁-C₆ alkyl* in the context of the present specification relates to a saturated or unsaturated linear or branched hydrocarbon having 1, 2, 3, 4, 5 or 6 carbon atoms. Non-limiting examples for a C₁-C₆ alkyl include methyl, ethyl, propyl, 1-methylethyl (isopropyl), n-butyl, 2-methylpropyl, *tert*-butyl, n-pentyl, 2-methylbutyl, 3-methylbutyl, 1,1-dimethylpropyl, 1,2-dimethylpropyl, n-hexyl, 3-methyl-2-pentyl, and 4-methyl-2-pentyl.

The term "signal enhanced compound" or "signal enhanced compound that comprises a nuclear spin" relates to a compound that comprises a hyperpolarized hydrogen atom or a hyperpolarized heteronucleus. The signal enhanced compound may be used as such as a contrast agent after purification, or may be modified before using the modified signal enhanced compound as contrast agent after purification. Modifications can be the transfer of the spin order or chemical transformations such a cleavage into a metabolite.

Contrast agent is the purified version of a signal-enhanced compound which is potentially intended to be supplied to a biological sample (enzyme, cells) or intended to be injected into an organism.

The term "heteronucleus" relates to a nucleus other than hydrogen. Most suitable heteronuclei are ¹³C or ¹⁵N, particularly ¹³C

The term "hyperpolarized H atom", "hyperpolarized ¹³C atom" or "hyperpolarized ¹⁵N atom" refers to a H atom, a ¹³C atom or a ¹⁵N atom comprising a hyperpolarized spin.

The term "liquid" relates to the liquid comprising the signal enhanced compound before the evaporation step. The liquid comprises additionally a neat catalyst, such as a ionic liquid, or a solvent and a catalyst, or a solvent and a source of radicals, whereby the solvent can be the signal enhanced compound. Furthermore, the liquid may contain by-products and/or unreacted/non-polarized educts and other impurities.

The signal enhanced compounds are not gaseous under the conditions prior to the evaporation step.

Unless defined otherwise, all technical and scientific terms used herein have the same meaning as commonly understood by one of ordinary skill in the art.

### Description

Contrast agents that are suitable for the use in a clinical setting for example for the diagnosis of a disease by MRI or in pre-clinical studies should ideally be free of reactants, by-products, and catalysts or sources of radicals and generally impurities. If the preparation has been performed in an organic solvent, the contrast agent may be transferred to an aqueous solution that can be injected into an organism or applied on cells or enzymes.

Contrast agents may be prepared by spontaneous reactions or transfers, pulsed NMR methods, pulsed polarization transfer, field cycling or methods that make use of radicals in combination with microwave radiation. Well-known methods include for example PHIP, SABRE or DNP.

PHIP is a hydrogenative procedure. Suitable educts contain an unsaturated bond that will be hydrogenated during the process in the presence of a metal catalyst. As a result, the educt changes its chemical structure during the hyperpolarization step. For example, a vinyl ester (educt) may be converted to a para-hydrogenated ethyl ester. The para-hydrogenated ethyl ester may be used as such as a contrast agent. Alternatively, the spin order of the para-hydrogenated ethyl ester may be further transferred to a heteronucleus such as ¹³C or ¹⁵N and subsequently used as contrast agent. Optionally, the ethyl ester may be cleaved. For instance, a hyperpolarized ethyl pyruvate may be cleaved to obtain hyperpolarized pyruvate.

To obtain a highly purified contrast agent, at least one purification step is required. The purification may be performed after the hydrogenation step and/or after the transfer of the spin order to a heteronucleus.

SABRE is a non-hydrogenative procedure. In contrast to PHIP, the parahydrogen does not react in an addition reaction but forms a temporarily stable complex with a catalyst and the educt (e.g. a metabolite such as pyruvate) in which the spin order of the parahydrogen is transferred to hydrogen atoms of the educt. The labile complex then separates in its components again. Hence, the educt does not change its chemical structure during the hyperpolarization step. The signal enhanced compound obtained may be used as such as contrast agent, or the spin order may optionally be further transferred to a heteronucleus such as ¹³C or ¹⁵N before using the signal enhanced compound as contrast agent. In specific cases of SABRE, the spin order of para-hydrogen is directly transferred to the spin of a heteronucleus.

A purification step may be performed after the transfer of the spin order of the parahydrogen to the educt and/or after the further transfer of the spin order to a heteronucleus.

Signal enhanced compounds may also be prepared by optimized PHIP or SABRE methods. Such method comprises the steps of
- providing a molecule comprising two protons and at least one heteronucleus (S₃, S₄), the protons having nuclear spins being coupled to a nuclear spin of the at least one heteronucleus;
- exposing the protons and the at least one heteronucleus to a magnetic field (*B*₀) in a z direction, the z direction forming a right-handed orthogonal coordinate system with an x and a y direction;
- applying a sequence of radio frequency pulses to the protons and the at least one heteronucleus in order to transfer said two-spin order into the hyperpolarization of the at least one heteronucleus, wherein said sequence of radio frequency pulses comprises a first, a second, and a third group (*N_{A}, N_{B}, N_{C}*) of 180° radio frequency pulses, wherein the first group (*N_{A}*) of 180° radio frequency pulses is consecutively applied *n_{A}* times during a first time interval (*τ_{A}*) and wherein the second group (*N_{B}*) of 180° radio frequency pulses is consecutively applied *n_{B}* times during a second time interval (*τ_{B}*) after the last first group, and wherein the third group (*N_{C}*) of 180° radio frequency pulses is consecutively applied *n_{C}* times during a third time interval (τ*_{C}*) after the last second group, wherein *n_{A}*, *n_{B}, n_{C}* are integer numbers, respectively.

A purification step may be performed after transfer of the two-spin order or longitudinal order. In DNP, the higher polarization of electron spins is converted in hyperpolarized nuclear spin. No para-hydrogen is used at all. Instead, typically radicals are used as a source of spin order to signal-enhance molecules of interest. As a result, the educt does not change its chemical structure during the hyperpolarization step.

After hyperpolarization, the signal enhanced compound may be purified.

The aim of the purification step is to separate the catalyst (PHIP, SABRE) or to separate the source of radicals (DNP) from the signal enhanced compound.

Typically, the proton longitudinal relaxation time of the signal enhanced compounds is in the range of 1 to 15 seconds and can be on the order of 60 seconds for e.g. ¹³C spins. To maintain the signal, the purification needs to be performed fast, i.e. in the range of seconds rather than minutes. Such fast purification is achieved by a quick evaporation step.

A first aspect of the invention relates to a method for preparing a contrast agent characterized in that the method comprises an evaporation step, wherein a signal enhanced compound that comprises a nuclear spin is separated from a liquid.

In certain embodiments, the contrast agent is prepared by PHIP (para-hydrogen induces polarization), by SABRE (signal amplification by reversible exchange), or by DNP (dynamic nuclear polarization).

The liquid relates to the liquid comprising the signal enhanced compound before the evaporation step. The liquid comprises
- a neat catalyst, such as a ionic liquid, and the signal enhanced compound, or
- a solvent, the signal enhanced compound and a catalyst, or
- a solvent, the signal enhanced compound and a source of radicals,
whereby the solvent can be the signal enhanced compound. Furthermore, the liquid may contain by-products and/or unreacted/non-polarized educts and other impurities.

When PHIP or SABRE are performed to prepare a signal enhanced compound, the liquid is a solution that comprises a solvent, the signal enhanced compound and a catalyst, whereby the solvent can be the signal enhanced compound..

When DNP is performed to prepare a signal enhanced compound, the liquid is a solution that comprises a solvent, the signal enhanced compound and a source of radicals, whereby the solvent can be the signal enhanced compound.

In certain embodiments, the liquid comprises the signal enhanced compound and a catalyst. The catalyst can be a solid catalyst dissolved in the signal enhanced compound. In certain embodiments, the liquid comprises the signal enhanced compound and a source of radicals.

Furthermore, the liquid may contain by-products and/or unreacted/non-polarized educts and other impurities

Depending on the boiling temperature, either solvents of the liquid are evaporated or the signal enhanced compound is evaporated. If the liquid comprises several components that differ in their boiling temperature, e.g. a solvent, the signal enhanced compound and a catalyst, more than one distillation may be required, for example first evaporating the organic solvent and then evaporating the signal enhanced compound. It could also be necessary to first evaporate unreacted precursor compounds before evaporating solvents and/or the signal enhanced compounds. Suitable solvents are e.g. aqueous solvents, methanol, chloroform or acetone. To allow a fast evaporation step, the components may be chosen in such a way that the boiling temperature of the signal enhanced compound is the lowest so that the signal enhanced compound can be distilled in one step from the liquid in the reaction vessel.

In certain embodiments, the liquid comprises a catalyst, the signal enhanced compound and an organic solvent that is immiscible with water. Prior to the evaporation water is added forming two phases. The signal enhanced compound is separated from the liquid by evaporating the organic solvent and transferring the signal enhanced compound into the aqueous phase providing purified signal enhanced compound in water.

Desired chemical transformation (e.g. cleavage) of the enhanced compound can occur during this process (e.g. if a cleaving agent such as base had been added to the water). Afterwards impurities can be filtered out from the aqueous solution.

In certain embodiments, the signal enhanced compound is separated from the liquid by evaporating the signal enhanced compound providing a purified signal enhanced compound. In certain embodiments, the signal enhanced compound is separated from the liquid by first evaporating an organic solvent and after separating the organic solvent the signal enhanced compound is evaporated providing a purified signal enhanced compound.

In certain embodiments, the signal enhanced compound is separated from the liquid by evaporating the signal enhanced compound providing a purified signal enhanced compound , wherein the purified signal enhanced compound is modified by the transfer of the spin order and/or chemical transformations such a cleavage into a metabolite, wherein the purification after the modification of the signal enhanced compound is achieved by an additional evaporation step of the signal enhanced compound.

Additional purification steps may be performed after any evaporation step such as filtration, precipitation and washing or liquid phase separation providing the contrast agent.

In certain embodiments, the signal enhanced compound is separated from the liquid by evaporating the signal enhanced compound providing a purified signal enhanced compound, wherein the purified signal enhanced compound is modified by the transfer of the spin order or chemical transformations such a cleavage into a metabolite, wherein the purification after the modification of the signal enhanced compound is achieved by an additional purification step such as filtration, precipitation and washing or liquid phase separation.

To maintain the spin order of the signal enhanced compound, the evaporation step may be performed in a static magnetic field.

In certain embodiments, the evaporation step is performed in a static magnetic field.

In certain embodiments, the evaporation step is performed in a static magnetic field having a magnetic field strength of at least 10 mT.

The longitudinal relaxation time depends on temperature and pressure. Relaxation times in the gas phase are typically effected by the spin rotation relaxation. For large molecules such as ethyl lactate, ethyl pyruvate or ethyl acetate, which are particularly suitable as either precursors for signal enhanced contrast agents or as contrast agents themselves (e.g. ethyl pyruvate), is on the order of 1-15 seconds in the gas phase. Longer times are preferred and they can be obtained by increasing the pressure and/or temperature in the vapor phase. A fast distillation step is hence feasible and can be used as an excellent mechanism during the purification to maintain the signal enhancement.

The evaporation may be further improved by using a co-solvent or a stripping gas.

In certain embodiments, a co-solvent or a stripping gas is used during the evaporation step.

In certain embodiments, a stripping gas is used during the evaporation step.

In certain embodiments, the signal enhanced compound is evaporated at a pressure of at least 3 bar.

In certain embodiments, the signal enhanced compound is evaporated under pressure, particularly at a pressure of at least 3 bar, and a stripping gas is used.

In certain embodiments, the signal enhanced compound is evaporated at a temperature of at least 390 K.

The time that is required to evaporate a certain amount of the signal enhanced compound may be reduced by applying vacuum during the evaporation step.

In certain embodiments, vacuum is applied during the evaporation step. The evaporation with or without stripping gas can be facilitated by applying a vacuum.

In certain embodiments, the evaporation step with a stripping gas is facilitated by applying a vacuum.

In certain embodiments, the signal enhanced compound is evaporated with a stripping gas under pressure, wherein the evaporation is facilitated by by applying a vacuum, in particular by applying 10 mbar or below.

In certain embodiments, the evaporation step is performed at 10 mbar or below.

In certain embodiments, the evaporation step is performed at 10 mbar or below and no stripping gas is applied.

Particularly when preparing the signal enhanced compound via PHIP, non-reacted unsaturated educts may still be present in the liquid. To prevent potential toxicity, the educts may be quenched. This can be performed e.g. by adding an excess of thiol containing biomolecules such as the amino acid cysteine that reacts with the unsaturated precursor in a thiol-ene reaction. The reaction product needs to be non-volatile which is typically accomplished when e.g. using the salt of the biomolecule. An additional catalyst may also be added or a radical needs to be introduced to speed up this process. It is however important that they are not volatile and remain in the reaction vessel while the signal enhanced compound is evaporated and transferred to another vessel.

In certain embodiments, prior to the evaporation step an unreacted educt in the liquid is converted to a non-volatile product, particularly to a non-volatile salt. This could be achieved for example by adding the amino acid cysteine or another suitable molecule in the presence of a catalyst to scavenge unreacted signal enhanced precursor by reacting with its double or triple C-C bond.

In certain embodiments, the purified signal enhanced compound
- is condensed out after evaporation and then dissolved in an aqueous solvent, or
- directly trapped in an aqueous solvent after evaporation.

The solvent in which the purified signal enhanced compound is dissolved or directly trapped may be a biocompatible solvent.

In certain embodiments, the signal enhanced compound is volatile.

In certain embodiments, a chelating molecule is added before the evaporation step binding the catalyst and making it even less volatile

To allow efficient distillation wherein only the signal enhanced compound is evaporated, there needs to be a sufficient difference in boiling temperature between the liquid and the signal enhanced compound.

In certain embodiments, the signal enhanced compound has a boiling temperature that differs from the liquid by at least 20 °C, particularly by at least 40 °C.

In certain embodiments, the signal enhanced compound comprises a hyperpolarized H atom or a hyperpolarized ¹³C atom or a hyperpolarized ¹⁵N atom.

Suitable signal enhanced compounds are hyperpolarized metabolites such as esters of amino acids that have a short side chain (e.g. alanine or serine), pyruvate or an alkylester thereof, lactate, acetate, succinate or an alkylester thereof, fumarate or an alkylester thereof, acetoacetate or an alkylester thereof, hydroxybutyrate or an alkylester thereof, ketoisocaproate or an alkylester thereof, malate or an alkylester thereof, citrate or an alkylester thereof. These signal enhanced compounds may be obtained by PHIP, SABRE or DNP.

When SABRE is performed for preparing a signal enhanced compound, heterocycles such as pyridine or nicotinamide may also be used as educts.

In certain embodiments, the signal enhanced compound is selected from a five- or six-membered heterocyle that comprises one or more N atoms, nicotinamide or a compound of formula I.

In certain embodiments, the signal enhanced compound is a compound of formula I, 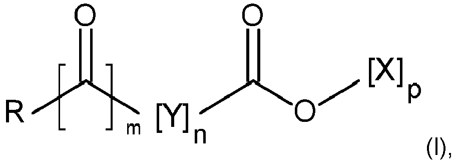 wherein
R is selected from H, -OH, -OX' and C₁₋₄-alkyl, wherein the alkyl is unsubstituted or substituted by -OH,
Y is a C₁₋₆-alkyl or a C₁₋₆-alkenyl, wherein the alkyl is unsubstituted or substituted by one or more substituents selected from NH₂, -OH, -COOH and -COOX",
X, X' and X' are independently of each other a C₁₋₄-alkyl,
m, n and p are independently of each other 0 or 1,
wherein the compound of formula I comprises at least one hyperpolarized atom, particularly a hyperpolarized H atom or a hyperpolarized ¹³C atom or a hyperpolarized ¹⁵N atom, wherein in particular one or more or all H can be exchange with deuterium.

When PHIP is performed to obtain a signal enhanced compound, the educt comprises a double bond. Suitable educts are compounds that comprise a vinyl, allyl or propargyl moiety. These moieties are para-hydrogenated during PHIP. The para-hydrogenated moiety corresponds to the moiety X.

In certain embodiments, X. X' and X" are ethyl, wherein each C atom is bound to a hyperpolarized H atom.

In certain embodiments, the contrast agent is selected from hyperpolarized pyruvate or an alkylester thereof, lactate, acetate, succinate or an alkylester thereof, fumarate or an alkylester thereof, acetoacetate or an alkylester thereof, hydroxybutyrate or an alkylester thereof, ketoisocaproate or an alkylester thereof, malate or an alkylester thereof, citrate or an alkylester thereof.

In certain embodiments, the contrast agent is selected from hyperpolarized pyruvate or an alkylester thereof, lactate, acetate, succinate or an alkylester thereof, fumarate or an alkylester thereof, acetoacetate or an alkylester thereof, hydroxybutyrate or an alkylester thereof.

In certain embodiments, the alkyl ester is a C₁₋₆-alkyl ester, particularly a C₁₋₄ alkyl ester, more particularly an ethyl ester.

If PHIP or SABRE is performed to obtain the signal enhanced compound, a catalyst is required for the addition of parahydrogen or the transfer of the spin order. Suitable catalysts are known to a skilled person.

In certain embodiments, the catalyst is selected from a rhodium catalyst, an iridium catalyst, a platinum catalyst, a palladium catalyst, ruthenium or rhenium catalyst.

A suitable rhodium catalyst is a Wilkinson catalyst or a rhodium complex that comprises phosphine ligands such as [1,4-Bis-(diphenylphosphino)-butan]-(1,5-cyclooctadien)-rhodium(l)-tetrafluoroborat.

A suitable ruthenium catalyst is a cyclopentadiene complex such as [Cp*Ru(MeCN)₃]PF₆ :Tris-(acetonitril)-pentamethylcyclopentadienylruthenium(II)-hexafluorophosphat.

An iridium catalyst may particularly be used for SABRE. A suitable catalyst is a Crabtree catalyst or an Ir complex with N-heterocyclic carbene ligands such as [IrCl(cod)(IMes)]; COD: cycloocatdiene; IMes: 1,3-Bis(2,4,6-trimethylphenyl)imidazoliniumchlorid.

If DNP is used for preparing the signal enhanced compound, a source of radicals is used. Suitable radicals are known to a skilled person. Non-limiting examples are nitroxide radicals such as TEMPO, AMUPOL and TOTAPOL, or trityl radicals such as Ox063.

The invention is further illustrated by the following examples and figures, from which further embodiments and advantages can be drawn. These examples are meant to illustrate the invention but not to limit its scope.

### Description of the Figures

Fig. 1 shows an example procedure to obtain signal-enhanced pyruvate as clean contrast agent.
Fig. 2 shows another example procedure to obtain signal-enhanced pyruvate as clean contrast agent.

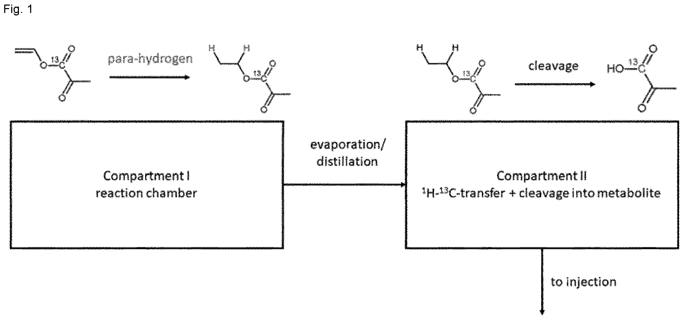

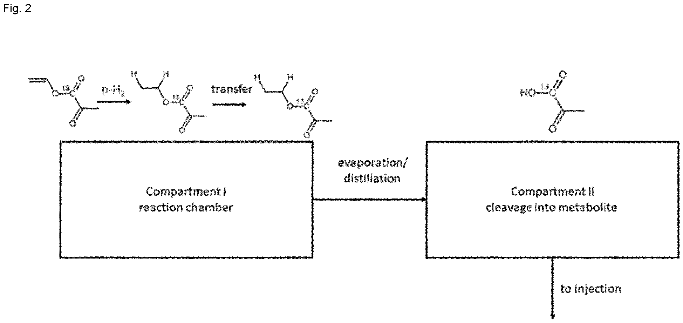

At first, a volatile precursor of the contrast agents is required which may also be the final desired molecule (e.g. ethyl pyruvate, ethyl lactate, ethyl acetate, diethyl fumarate, ethyl acetoacetate or ethyl hydroxybutyrate). The latter is usually the case when SABRE is used. The metabolite of interest or a precursor thereof is then reacted in a solvent or as neat compound with a catalyst and para-hydrogen inside a first compartment (reaction chamber). See Figure 1 and 2 for details on example procedures. If a solvent is used this is preferably water to circumvent the use of organic solutions or solvent with boiling point that differs a lot from those of the metabolite or its precursor. After the reaction, the compound is separated from the solvent by distillation and collected in a second compartment. Depending on boiling points of the metabolite and the solvent, the compound is distilled from solvent or first the solvent is distilled out and in the second step the compound is distilled.

The metabolite is either condensed out and then taken up by a biocompatible solvent (water, buffer, saline solution etc) or directly trapped in an aqueous medium. A stripping gas may be used in addition or a co-solvent for co-evaporation that however needs to be removed before in vivo application (by potentially further evaporation/distillation after conversion of a precursor into the desired contrast agent). To prevent the catalyst from partial distillation a heavy chelate molecule can be added into solution to bind the metal before the distillation starts. Further distillation may also be required if the original reaction in the first compartment is performed in an organic solvent or it is necessary to remove unreacted precursor molecules. Basic cleavage of the precursor and neutralization can occur in the second compartment.

### Examples

### Example 1:

A concrete implementation of the invention can be to place the first compartment in a low magnetic field, which may be the earth's field. The reaction of neat contrast agent precursor with parahydrogen is carried out in said low field and the contrast agent evaporated and transported into the second compartment which is placed inside a higher field magnet. The higher magnetic field can e.g. have from 100 mT to several Tesla field strength. The magnetic field in the high field magnet is high enough that the chemical shift in hertz should be larger than the J-coupling among the two parahydrogen stemming protons. At this higher magnetic field pulse sequences can be used to obtain in-phase proton magnetization. In case that contrast agents are used in which heteronuclei (nuclei other than protons such as ¹³C or ¹⁵N) are signal-enhanced, pulse sequences need to be applicable to transfer the proton polarization to said heteronucleus. This poses less requirements for the homogeneity of the higher magnetic field. The contrast agent is then ejected or washed out by physiological solution from this chamber and can be used for administration.

### Example 1a:

Example 1a is as Example 1 whereby the reaction is carried out in the first compartment that is located in a higher field ranging from 100 mT to several Tesla. The preparation of the in-phase proton magnetization or polarization transfer to the heteronuclei is performed in the same compartment. The contrast agent is distilled into the second compartment.

### Example 2:

Example 2 is as Example 1 and 1a whereby the contrast agent precursor is dissolved in a solvent with a higher boiling point than contrast agent. After hydrogenation, the contrast agent is evaporated from the solvent into the second chamber. Preferably the difference in boiling point between the contrast agent and the solvent is >20°C, more preferable >40°C.

### Example 3:

Example 3 is as Example 1 and 1a whereby the contrast agent precursor is dissolved in a solvent with a lower boiling point than contrast agent. After hydrogenation the separation is conducted in two steps. During the first step, the temperature of both chambers is set to or less than the boiling point of the solvent and the solvent is evaporated. In the second step the temperature of the first chamber is set higher or close to the contrast agent's boiling point and the contrast agent is then distilled into the second chamber. Preferably the difference between contrast agent and solvent is >20°C, more preferable >40°C.

### Example 4:

Example 4 is as Examples 1,1a,2,3 whereby after the hydrogenation, the evaporation is facilitated by a carrier/stripping gas flow through the first chamber. For the same purpose a vacuum can be applied during distillation.

### Example 5:

Example 5 is as Examples 1,1a,2,3,4 where a chelating molecule is added to the reaction mixture after the hydrogenation to bind the catalyst and make it even less volatile.

### Example 6:

Example 6 is as Examples 1,1a,2,3,4,5 whereby the amino acid cysteine or another suitable molecule is added in the presence of a catalyst to scavenge unreacted contrast agent precursor by reacting with its double or triple C-C bond and make them non-volatile.

### Example 6a:

Example 6a is as Examples 1,1a,2,3,4,5 where contrast agent precursor is a vinyl compound (e.g. vinyl pyruvate). The non-hydrogenated vinyl precursor is cleaned from the product contrast agent by distilling it out because it has a lower boiling point than the product molecule. In Example 1, 1a, 2 an additional distillation step before distilling the product is required. While in Example 3 the vinyl compound is distilled together with solvent.

### Example 7:

Example 7 is as the examples above whereby the actual contrast agent is obtained by cleavage of the distilled product after the hydrogenation reaction in the second compartment and after a pulse sequence has already been applied. The final contrast agent is obtained in the second compartment. The contrast agent can be purified from cleaved waste by distilling it out as presented in examples 3 and 4 for the first compartment.

### Example 8:

Example 8 is as Example 7 whereby the distilled molecule in the second compartment is dissolved in an amphiphilic solvent (e.g. acetone, alcohol) to facilitate the cleavage reaction.

### Example 9:

Example 9 is as Examples 7,8 when the molecule requires a two-stage cleavage. First the anhydrous base for cleavage in an appropriate solvent (e.g. alcohol) is added and after several seconds water is added.

### Example 10:

The precursor molecule with catalyst is para-hydrogenated in organic solvent that is immiscible with water. Afterwards the water is added and the mixture is vigorously mixed or ultrasound is applied. The organic solvent is evaporated as in Examples 3,4 and the enhanced molecule is transferred to the aqueous phase. Afterwards the remaining catalyst is filtered and the solution ready for administering.

### Example 11

Same as Example 10 where the precursor is vinyl pyruvate and instead of water, a solution of carbonate or sodium hydroxide is added. In the end a cleaved aqueous solution of pyruvate is obtained.

## Claims

1. A method for preparing a contrast agent
**characterized in that**
the method comprises an evaporation step, wherein a signal enhanced compound that comprises a nuclear spin is separated from a liquid.

2. The method according to claim 1, wherein the contrast agent is prepared by PHIP (para-hydrogen induced polarization), by SABRE (signal amplification by reversible exchange), or by DNP (dynamic nuclear polarization).

3. The method according to one of the preceding claims, wherein the liquid comprises a catalyst or a source of radicals.

4. The method according to one of the preceding claims, wherein the signal enhanced compound is separated from the liquid by evaporating the signal enhanced compound providing a purified signal enhanced compound.

5. The method according to one of the preceding claims, wherein the evaporation step is performed in a static magnetic field, particularly in a static magnetic field having a magnetic field strength of at least 10 mT.

6. The method according to one of the preceding claims, wherein the signal enhanced compound is evaporated at a pressure of at least 3 bar and/or at a temperature of at least 390 K.

7. The method according to one of the preceding claims, wherein a co-solvent or a stripping gas is used during the evaporation step, particularly a stripping gas is used during the evaporation step.

8. The method according to one of the preceding claims, wherein vacuum is applied during the evaporation step, particularly vacuum of 10 mbar is applied.

9. The method according to one of the preceding claims, wherein prior to the evaporation step an unreacted educt in the liquid is converted to a non-volatile product, particularly to a non-volatile salt.

10. The method according to claim 4, wherein the purified signal enhanced compound
- is condensed out after evaporation and then dissolved in an aqueous solvent, or
- directly trapped in an aqueous solvent after evaporation.

11. The method according to one of the preceding claims, wherein the signal enhanced compound is volatile.

12. The method according to one of the preceding claims, wherein the signal enhanced compound has a boiling temperature that differs from the liquid by at least 20 °C, particularly by at least 40 °C.

13. The method according to one of the preceding claims, wherein the signal enhanced compound comprises a hyperpolarized H atom or a hyperpolarized ¹³C atom or a hyperpolarized ¹⁵N atom.

14. The method according to one of the preceding claims, wherein the signal enhanced compound is selected from a five- or six-membered heterocyle that comprises one or more N atoms, nicotinamide or a compound of formula I, particularly a compound of formula I, 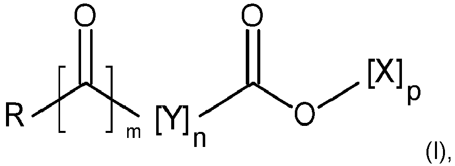 wherein
R is selected from H, -OH, -OX' or C₁₋₄-alkyl, wherein the alkyl is unsubstituted or substituted by -OH,
Y is a C₁₋₆-alkyl or a C₁₋₆-alkenyl, wherein the alkyl is unsubstituted or substituted by one or more substituents selected from NH₂, -OH, -COOH or -COOX',
X, X' and X" are independently of each other a C₁₋₄-alkyl, in particular X, X' and X" are ethyl, wherein each C atom is bound to a hyperpolarized H atom,
m, n and p are independently of each other 0 or 1,
wherein the compound of formula I comprises at least one hyperpolarized atom, particularly a hyperpolarized H atom or a hyperpolarized ¹³C atom or a hyperpolarized ¹⁵N atom, wherein in particular one or more or all H can be exchanged with deuterium.

15. The method according to one of the preceding claims, wherein the contrast agent is selected from hyperpolarized pyruvate or an alkylester thereof, lactate, acetate, succinate or an alkylester thereof, fumarate or an alkylester thereof, acetoacetate or an alkylester thereof, hydroxybutyrate or an alkylester thereof, ketoisocaproate or an alkylester thereof, malate or an alkylester thereof, citrate or an alkylester thereof and/or the catalyst is selected from a rhodium catalyst, an iridium catalyst, a platinum catalyst, a palladium catalyst, a ruthenium catalyst or a rhenium catalyst.
